# EUROPEAN PATENT SPECIFICATION

(11) **EP 2 606 885 B1**
(45) Date of publication and mention of the grant of the patent: **11.05.2016**
(21) Application number: 11865767.5
(22) Date of filing: 10.11.2011
(51) Int. Cl.: A61K 31/375, A61K 31/70, A61K 33/14, A61K 33/18, A61P 1/04, A61P 31/04, A61P 31/12, A61P 37/04

(54) **MEDICINAL PREPARATION "RENESSANS" HAVING AN ANTIBACTERIAL, ANTI-ULCEROUS AND IMMUNO-MODULATING ACTION**
WIEDERHERSTELLUNG EINES ARZNEIMITTELPRÄPARATS MIT ANTIBAKTERIELLER, ANTIULZERÖSER UND IMMUNMODULIERENDER WIRKUNG
PRÉPARATION MÉDICINALE "RENESSANS" À ACTION ANTIBACTÉRIENNE, ANTI-ULCÈRE ET IMMUNOMODULATRICE

(30) Priority: 16.05.2011 KZ 20110501
(43) Date of publication of application: 26.06.2013
(73) Proprietor: Begaliev, Shokan Sabirkhanovich, Almaty (KZ); Nugerbekova, Kulshat Magzumovna, Astana (KZ)
(72) Inventor: Begaliev, Shokan Sabirkhanovich, Almaty (KZ); Nugerbekova, Kulshat Magzumovna, Astana (KZ)
(74) Representative: Jeck, Anton
(86) International application number: PCT/KZ2011/000018
(87) International publication number: WO 2012/158002

(56) References cited:
- WO-A1-01/78751
- WO-A1-99/65538
- KZ-A- 13 765
- KZ-B- 6 730
- US-A- 5 955 101
- US-A1- 2003 211 173
- BASHKIRTSEVA N.: 'Cindii yod- i nedug yudet.' IZD-VO ''KRYLOV'', [Online] 2009, Retrieved from the Internet: <URL:http://yandex.ru/yandsearch?text=%D0%9 1 %DO%90%DO%A8% DO%9A%DO%98%..> [retrieved on 2012-01-11]
- 'loda spirtovoy rastvor - Instruktsiya, primenenie, tsena, otzyvy.lstochniki dannykh.' ENTSIKOPEDIYA LEKARSTV, 2004, M.D. MASHKOVSKII. LEKARSTVENNYE SDREDSTVA, [Online] 2000, Retrieved from the Internet: <URL:http://poisklekarstva.ru/medlist.php?i d=3848> [retrieved on 2012-01-26]

## Description

The invention relates to the pharmaceutical industry, in particular to the preparation of iodine-containing pharmaceuticals.

Nowadays, iodine medications play an important role as modern antiseptics. Pathogens of many infections are sensitive to iodine-containing medications, which exhibit fungicidal, cellulicidal and antiprotozoal properties, active against some spores and cause no acquired resistance.

The problem with using iodine is that in high concentrations it is toxic, irritating to skin and mucous membranes. However, in combination with polysaccharides and polymers, iodine largely loses its toxicity, exhibits slower and prolonged action (over 5 hours), while maintaining antiviral and antimicrobial properties (Nikulin V., V. Gerasimenko, 2008; E. Svirskaya, 2008).

Iodine also exhibits fungicidal, cellulicidal and antiprotozoal properties and somewhat active against some spores. During resorption, it is actively involved in the metabolic processes of the body increasing the lecithin-cholesterol ratio, lipoprotein and fibrinolytic activity of the blood; slowing blood clotting: lowering cholesterol and β - lipoproteins.

Complexes of iodine with polymers (iodinolum, iodonatum, iodovidonum, povidoneiodine, iodopiron, iodophor, hiniofon) are used as antiseptics for the ablation of tonsils in chronic tonsillitis, suppurative otitis media, sinusitis, keratitis, furunculosis, periodontitis, conjunctivitis, and for treatment of wounds and the operative field.

It is known that povidone-iodine (iodine in the form of a complex with the binding iodophor - polyvinylpyrrolidone) has a wider spectrum of antibacterial and antiviral properties than other known antiseptics. It is potent against many kinds of viruses, including HIV, in the suspension experiments in vitro [Kawana R. et.al., Inactivation of human viruses by povidon-iodine in comparison with other antiseptics. - Dermatology, 1997,195, Appendix 2, pages 29-35]. Anti-infective pharmaceuticals containing povidone-iodine can be used to fight diseases caused by HIV, chlamydia, gonococcus, treponema and herpes simplex virus [Tsutomu S., Junko S. Anti-infective against STD relating, for example, to HIV, chlamydia, gonococcus, treponema palladium or herpes simplex and device used to preserve/mix principal ingredient and base of such anti-infective - EP 0823996A1, A 01N 59/12, A 51 K 9/00, applic. 15.08.96, publ. 18.02.98 and Shanbrom E.Antiviral,spermicidal vaginal gel and foam containing low molecular weight povidoneiodine. US 5545401, A 61K 31/79, A 61K 33/18, applic. 02.06.94, publ. 13.08.96].

Iodomidolum is known as a medical preparation with antiviral and antibacterial properties [patent number of RK 6730, applic. 26.01.96, publ. 11/16/98], which contains iodine, potassium or sodium iodide, a synthetic water-soluble polymer acting as a polymer matrix, a mixture of natural polymers, such as poly-, mono-and oligosaccharides, as well as water in the following ratio, g / L: Iodine 6 - 10, potassium or sodium iodide 9-15, synthetic water-soluble polymer 2-4, mono-, oligo-and polysaccharides 8-120, water and the rest.

This medical preparation has relatively high toxicity and is intended for the treatment of viral and other bacterial diseases of animals.

A therapeutic medical preparation that has bactericidal, viricidal activity and contains iodine, potassium or sodium iodide, a synthetic water-soluble polymer, natural polymers, such as polysaccharides, in the following ratio, g / l: 6 -10 iodine, potassium or sodium iodide 9 - 15, a synthetic water-soluble polymer 2 - 4, natural polymers (polysaccharides) and mono-and oligosaccharides 8 - 120, water and the rest (CZ 5435, A 61 K 33/18, A 61 K 31/715) is also known.

The disadvantage of this medical preparation is its relatively high toxicity. To affect the viruses that have already penetrated the cells is difficult, because the cell itself protects them from any impact. It is the adapted virus that is destroying the cell. The objective is to suppress specific processes of the biological synthesis of viral particles on the molecular level possibly without any harm to the vital activity of the cells uninfected by the virus.

US 2003/211173 A1 describes an aqueous lubricating gel for use in mucosal areas of the human body having improved antimicrobial activity comprising at least 30 ppm of available iodine, potassium iodide and ascorbic acid.

WO 01/78751 A1 discloses an antiviral and antibacterial pharmaceutical preparation containing 0.8-25% of iodine, 1.2-38% of potassium iodide and 9% of sodium chloride in water, which is administered intravenously.

WO99/65538 A1 describes an anti-infective medical device comprising a polymeric matrix containing glucose with an oxidant producing iodine component. The medical device is designed for the prevention of chronic acquired infections associated with insertion into body cavities. WO99/65538 A1 describes the coating of plastic implant devices with layers of elemental iodine-polymer dissolved in a polymer base in such a manner so as to cause adherence of the iodine-polymer on the surface of the implant device.

The closest technical substance is the medical preparation, balsam "Vozrozhdenie" (provisional patent of RK, number 13765, cl. A61K 33/18, 01.10.2003).

Balsam "Vozrozhdenie " contains (wt %) 0.5 - 1.5 iodine, 1.5 - 1.7 potassium iodide, 14.0 - 16.0 starch, 2.0 - 3.0 glucose, 8.0 - 10 glycerol, 2.0 - 3.0 lactic acid, 0.8 - 1.0 sodium chloride, 2.0 - 3.0 apple cider vinegar and the rest is distilled water.

However, balsam "Vozrozhdenie" is a dietary supplement, with no bactericidal activity against microbacteria and low bactericidal activity against spores, spore-forming bacteria and fungi. Balsam is designed for oral use only and does not allow administration of a pharmaceutical composition to people by intravenous or intramuscular, which in turn limits the range of usage.

The goal of the invention is to provide a medical preparation with antibacterial, antiulcer and immunomodulatory properties with low toxicity and possibility of its use in an injectable form, and also a method of obtaining such preparation.

A technical result of the present invention is expanding the range of medical preparations with antibacterial, antiulcer and immunomodulatory properties with low toxicity and possibility of its use in an injectable form.

The technical result is achieved with the medical preparation containing iodine, potassium iodide, starch, ascorbic acid, glucose, sodium chloride and purified water in the following ratio (wt %):

| | |
|---|---|
| Iodine | 0.4 - 2.0 |
| Potassium Iodide | 0.8 - 4.0 |
| Starch | 10.0 - 40.0 |
| Ascorbic acid | 0.4 - 2.0 |
| Glucose | 1.2 - 4.8 |
| Sodium chloride | 0.3 - 1.8 |
| Purified water | the rest |

It was unexpectedly found that the change in the composition of components, namely iodine, potassium iodide, starch, ascorbic acid, glucose and sodium chloride in the aqueous solution provides a medical preparation suitable for use in an injectable form. It contains fewer components, namely - 6 relative to the prototype, which included 9 components. The feature of the claimed composition that makes it distinct from the prototype is the composition and concentration of the components. The composition not containing two acid components - lactic acid and apple cider vinegar, and the triatomic alcohol glycerin and containing (in wt. %): iodine - 0.4 - 2.0; potassium iodide - 0.8 - 4.0; starch - 10.0 - 40.0; ascorbic acid - 0.4 - 2.0; glucose - 1.2 - 4.8; sodium chloride - 0.3 - 1.8 is the optimal composition for forming a balanced system of the components stable in an aqueous solution.

For the formation of the medical preparation as an integral substance modern principles of combinatorial chemistry were applied. One of the main functions of the components used in the composition of the medical preparation is forming a special structure that maintains the oxidizing power of iodine making it capable of transporting these particles over long distances, ultimately spreading over the whole body.

One of the main functions of the components used in this preparation is to form a special structure that maintains the oxidizing power of iodine making it capable of transporting these particles over long distances, ultimately spreading over the whole body. The formation of this system provides the optimum composition in the following ratio: iodine - 0.4 - 2.0; potassium iodide - 0.8 - 4.0; starch - 10.0 - 40.0; ascorbic acid - 0.4 - 2.0; glucose - 1.2 - 4.8; sodium chloride = 0.3 -1.8, purified water - the rest.

The specified composition and definite concentration of the components in an aqueous solution form an iodine-polymer complex that serves as a matrix and forms associates, from which the active agent, an active form of iodine, is released. Starch and glucose take part in the formation of the matrix and associates, that is poly- and monosaccharides with different activity of functional groups. The introduction of potassium iodide into the medical preparation facilitates forming a physical and chemical system from preventing deactivation of the main active agent for a long time. Nowadays it is known that carbohydrates are essential components of bacterial cell walls, being first to come into contact with the host's immune system. Due to this fact, the mechanisms of innate immunity include identification steps of bacterial carbohydrates and glycoconjugates associated with the function of a number of cellular receptors such as lectin, which have been recently observed on the surface of phagocytic cells.

When mixing starch with water, even with hot water, starch granules swell and only a small part of starch polysaccharides dissolve. A colloidal starch solution (starch paste) is prepared by stirring the starch in a small amount of water and gradually pouring the resulting mix into boiling water with vigorous stirring.

According to the generally accepted view, the formation of the amylose-iodine complex is associated with the helical conformation of amylose chains, the center of said helix forms a channel-like cavity sufficiently large to accommodate iodine molecules. The starch-iodine complex forms hexagonal crystals. It was found that the formation of the iodine complex does not change the viscosity of amylose solutions.

The study of the starch-iodine complex structure revealed it to be an inclusion complex with the donor-acceptor bond and valence, determined by the ratio of one iodine atom to eight monomer units of the polymer with the charge of the iodine component equal to zero. In the presence of starch, molecules J₂ break down with the formation of complex ions J₃ and JO, and the formation of the iodide-starch complex involves only the complex anion J₃, which disappears from the solution. Complex anion JO, on the contrary, remains in the solution, not entering into the complex. Its concentration increases sharply due to the splitting of J₂.

The study of the iodine-starch reaction provides a basis to present the mechanism thereof in the following form:

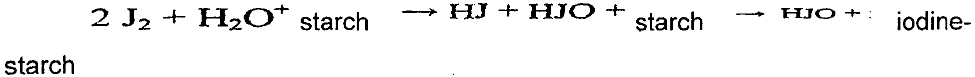

The medical preparation components promote selective oxidation of the vital molecular components of viruses and bacteria mostly by the effect of the active agent, iodine.

Thus, the components of the claimed preparation seek to maintain its stability and structuredness, and they are responsible for the direct manifestation of the antimicrobial properties.

All in all, the essential features of the medical preparation «RENESSANS" ensure the circulation of the medical preparation in the body as an active ingredient allowing its use in an injective form.

The preparation method of the medical preparation «RENESSANS" is as follows: the solutions of crystalline iodine and potassium iodide, sodium chloride and ascorbic acid in distilled water as well as a suspension of starch in water are prepared. All solutions are mixed and made up to 1 liter of water.

### Information supporting the possibility of the realization of the invention

An example of a preparation method of the medical preparation.

10 g of crystal iodine are ground and 16 g of potassium iodide are dissolved in a small amount of purified water. To make a slurry, purified water is added to 150 g of starch. Solutions of sodium chloride (9,0 g) and ascorbic acid (10.0 g) are prepared and everything is mixed and made up to one liter with purified water. The mixed medical preparation is in a syrupy liquid form, black to dark blue in color, with odor characteristic of iodine.

Examples confirming the properties and usability of the medical preparation «RENESSANS."

To examine the safety of the medical preparation «RENESSANS," it was administered to animals intraperitoneally in various doses, undiluted or diluted in a saline solution. The doses were selected based on the recommendations for the maximum allowable amount of fluid for injection into the peritoneal cavity, which for rats is 5 ml. The choice of the dose for injection was determined according to the classification of toxicity of chemicals in accordance with GOST 12.1.007-76. The medical preparation was administered intraperitoneally once daily.

During the experiment, the rats were divided into control and experimental groups. There were four experimental groups with different doses and timing of the administration.

The medical preparation dissolved in saline solution, and also undiluted was administered intraperitoneally to the animals.
1. The animals of the first group were injected intraperitoneally daily with 0.06 ml of the medical preparation + 3.0 ml of a saline solution, which exceeds the recommended therapeutic dose 1.7 times. The rats were sacrificed after 5, 10 and 15 days after the beginning of the experiment.
2. The animals of the second group were injected once with 0.66 ml of the medical, preparation and 3.0 ml of a saline solution, which is 11 times higher than the recommended therapeutic dose, the animals were sacrificed 5 days after the start of the administration.
3. The animals of the third group were injected once with 2 ml of the preparation and 3.0 ml of a saline solution, which is 33 times higher than the recommended therapeutic dose and the animals were sacrificed 10 days after the beginning of the experiment.
4. The fourth group of animals was injected once with 5 ml of pure medical preparation without dilution, which exceeds the recommended therapeutic dose 83 times and the animals were sacrificed 15 days after the beginning of the experiment.

The main data of the analysis of the morphological blood composition and differential white blood cell count are shown in Table 1.

As Table 1 shows, the investigated preparation "RENESSANS" did not cause statistically significant changes in the blood count of the experimental animals in comparison to the control rats, therefore, the investigated medical preparation had no toxic effects on the blood system. Histological examinations of the organs of animals in the control and experimental groups showed that the medical preparation «RENESSANS" did not cause any changes in the histological structure of the internal organs of the experimental animals, which also confirms non-toxicity of the medical preparation.

Investigation of the antimicrobial action of the medical preparation was conducted in vitro by the double dilution method in culture fluid.

The iodide starch and iodine-amylose solutions were prepared with the amount of potassium polyiodide calculated for 0.2 - 1 % solutions of polysaccharides.

The concentration of molecular iodine in solutions ranged from 0.27 to 1 %, the concentration of iodine polysaccharide was about 1 %.

In order to study the antibacterial effect of the medical preparation, the initial solutions were diluted with saline solution.

A bacterial suspension in a saline solution in the amount of 5 million cells, prepared on the daily agar culture of the tested strain were added to each dilution of iodine polysaccharide.

After a 15-minute exposure, each dilution of the medical preparation was plated in plain agar in Petri dishes.

The results were determined after 24 hrs. by the absence of the growth of bacteria, indicating full bactericidal activity in the high polymer with iodine solution.

The results are presented in Table 2.

Studies conducted to examine the efficacy of the medical preparation «RENESSANS" on 10 patients with gastric and duodenal ulcers over a 2-week period showed a high antiulcer effect of the medical preparation. Thus, during monotherapy with "RENESSANS", ulcer healing was observed in 9 cases regardless of their location (4 - gastric ulcer: mediogastric and antral and 5 DNA ulcers). Only in one case, the medical preparation caused increased pain (DNA ulcer), which required administration of an injection of antiinflammatory medical preparation. A follow-up esophagogastroscopy showed complete healing of ulcers in all 9 patients, and the pain subsided on the 1st - 2nd day of taking the medical preparation. In all cases, no correlation was found between the healing effect of the medical preparation and the size of the ulcers, age and sex of the patients. These data show high antiulcer efficacy of "RENESSANS" and attributed to its reparative properties. The immune status of 13 volunteers who had been receiving the medical preparation for 27 days was assessed twice (prior to the administration and after 27 days of the treatment). The studies were conducted at the Laboratory of Immunology of the Medical Center "Sunkar", licensed by the Almaty City Department of Health to perform clinical and immunological studies.

Evaluation of the immune status included: the total white blood cell count in the peripheral (venous) blood, lymphocytes, differential white blood cell count, relative and absolute content of major subpopulations of lymphocytes (total T-lymphocytes, their helper and suppressor subsets of B- lymphocytes, natural killer cells), nonspecific functional activity of T-lymphocytes, levels of the main classes of serum immunoglobulins and circulating immune complexes, phagocytal and functional-metabolic activity of neutrophils). The main lymphocyte subpopulations were identified on commercial kits of monoclonal antibodies to clusters CB3+, CB4+, CB8+-, CB16+, CB72+ (Russian-made) by indirect immunofluorescence on the microscope "LUMAM I-3": nonspecific functional activity of T-cells was studied by the direct leukocyte migration inhibition test (LMIT) for cell stimulation with PHA-P; concentration of the major classes of serum immunoglobulins (M, G, A) was identified by the traditional method of immunodiffusion in agar using commercial sets of monospecific antiimmunoglobulin sera: concentration of the total IgE by ELISA: circulating immune complexes - by the precipitation reaction in PEG-6000 followed by spectrophotometry of the precipitates: phagocytic activity of neutrophils was studied in the reaction of phagocytosis with latex particles and testing the nitroblue tetrazolium recovery (NBT test ), while both spontaneous and pyrogenal-stimulated parallel versions of both tests were carried out (Table 3).

Thus, the medical preparation «RENESSANS" can be used both for oral ingestion and for injections. The antimicrobial effect was found to be not only comparable to the aqueous solutions of iodine but in some cases exceeds them.

Optimal dosage forms of the medical preparation «RENESSANS" are suspensions for oral use and suspensions for injection. The "RENESSANS" preparation is nontoxic, has antimicrobial activity and an antiulcer effect.

Thus, a composition of a high polymer with iodine that was given the conventional name "RENESSANS," was developed as the result of the research. Given the wide range of pharmacological activity of the preparations containing high polymer with iodine, we developed a technology for production of the medical preparation «RENESSANS" in two dosage forms: suspensions for oral use and suspensions for injection. The flow sheet of the production of the medical preparation «RENESSANS" in the form of a suspension for oral use and a suspension for injection are shown in Figures 1 and 2.

The standardization and shelf life studies.

### "RENESSANS" preparation, suspension for oral use.

*Description.* Syrupy liquid dark blue to black in color with a distinctive taste and odor characteristic of iodine.
*pH.* From 3.0 to 5.0 (potentiometric, GF XI, ed. 1, p.113).
*Dry residue.* No less than 9.0 % (GF XI, ed. 2, page 148).
*Density.* 1.03 - 1.08 g/cm³ (GF XI, ed. 1, page 24).

*Microbiological purity.* Should not contain bacteria of the Enterobacteliaceae, Pseudomonas aeruginosa and Stphylococcus aureus families.

### "RENESSANS" preparation, suspension for injection.

*Description.* Syrupy liquid dark blue to black in color, with a distinctive taste and odor characteristic of iodine.

*Identification.* 5 ml of 0.1 M sodium thiosulfate was added to 5 ml of the medical preparation; solution is instantly decolorized (iodine).
Ph. From 3.0 to 5.0 (potentiometric, GF XI, Ed. 1, p.113).
*Dry residue.* No less than 9.0 % (GF XI, Ed. 2, page 148).
*Density.* 1.03 -1.08 g/cm³ (GF XI, ed. 1, page 24).

*Sterility.* The preparation is sterile. The tests conducted by direct plating in accordance with GF XI, Ed. 2, page 187.

*Pyrogenicity.* The preparation is apyrogenic. A test dose of 0.1 mg of the preparation dissolved in 1 ml of sterile water per 1 kg of the animal's body weight intravenously (GF XI, Ed. 2, p.183).

*Toxicity.* The preparation is non-toxic. A test dose is 0.1 mg of the preparation dissolved in 0.5 ml of water for injection, intravenous (GF XI, Ed. 2, p.182). Observation time is 48 hours.

Stability testing of the medical preparation "RENESSANS", suspensions for oral use, in accordance with the Guidelines CMP/ICH/2736/99 "Stability Testing of New Drugs and Products".
Stability test results for suspension of the medical preparation «RENESSANS" and suspension for injection medical preparation «RENESSANS" are given in Tables 1 and 2, respectively.

Thus, the results of the standardization study of the medical preparation «RENESSANS" for physical and chemical parameters of suspension for oral use and suspension for injection showed that the immunomodulatory medical preparation conforms to regulatory requirements.

Expiration date of the medical preparation for intravenous use and for injection were set based on all the parameters of quality and quantity, the expiration date for both medical preparations is 2 years.

### The study of the specific biological activity and safety of the medical preparation "RENESSANS".

The study of antimicrobial activity of the medical preparation was carried out in vitro by the double dilution method in culture fluid.

Starch iodide and iodine amilose solutions are prepared in the reaction of the calculated amount of potassium polyiodide with 0.2 - 1 % solutions of polysaccharides. The concentration of molecular iodine in the solutions ranged from 0.27 to 1 %, the concentration of iodine polysaccharide was about 1 %. To study the antibacterial properties of the medical preparation, stock solutions were diluted with saline solution. A suspension of bacteria in saline solution in the amount of 5 million cells prepared on a daily agar culture of the tested strain was added to each dilution of iodine polysaccharide.

After a 15-minute exposure, each dilution of the medical preparation was plated on plain agar in Petri dishes.

The results were determined after 24 hrs. by the absence of bacterial growth, indicating full bactericidal activity of the solution of the medical preparation "RENESSANS".

The results of these reference examples are presented in Table 1 and Table 2.

**Table 1**

| Test-microbe | Iodine concentration at which microbial growth is completely absent, y / ml | | |
|---|---|---|---|
| | Iodine starch | Iodine amilose | "RENESSANS" |
| Staph.aureus 209-p | 3-10 | 3-5.4 | 2-3.6 |
| Bac.subtilis 6633 ATCC | 5-10 | 3.7 | 1.4-2.7 |
| Esch.coli 25922 ATCC | 10 | 2.4 | 1.6 |
| Proteus vulgaris 6896 ATCC | 10-20 | 6.7 | 4.6 |
| Ps.aeroginosa 27853 ATCC | 10 | 5.8 | 2.7 |
| Microsporum canis 3/84 | 20 | 6.5 | 3.4 |
| Trichophyton gypscum 5.85 | 10 | 10 | 10 |
| Candida albicaps 1755 | 10 | 10 | 10 |

Studies conducted to examine the antiulcer efficacy of the medical preparation "RENESSANS" on 10 patients with gastric and duodenal ulcers over a 2-week period showed a high antiulcer effect of the medical preparation. Thus, during monotherapy with "RENESSANS," ulcer healing was observed in 9 cases, regardless of their location (4 - gastric ulcer: mediogastric and antral and 5 DNA ulcers). Only in one case the medical preparation caused increased pain (DNA ulcer) which required administration of an injection of an antiinflammatory medical preparation. A follow-up esophagogastroscopy showed complete healing of ulcers of all 9 patients, and the pain subsided on the 1st - 2nd day of taking the medical preparation. In all cases, no correlation was found between the healing effect of the medical preparation and the size of ulcers, age and gender of patients. These data show a high antiulcer efficacy of "RENESSANS" and attributed to its reparative properties. The immune status of 13 volunteers who had been receiving the medical preparation for 27 days was assessed twice (prior to the administration and after 27 days of the treatment). The studies were conducted at the Laboratory of Immunology of the Medical Center "Sunkar", licensed by the Almaty City Department of Health to perform clinical and immunological studies.

Evaluation of the immune status included: the total white blood cell count in the peripheral (venous) blood, lymphocytes, differential white blood cell count, relative and absolute content of major subpopulations of lymphocytes (total T-lymphocytes, their helper and suppressor subsets of B- lymphocytes, natural killer cells), nonspecific functional activity of T-lymphocyte, levels of the main classes of serum immunoglobulins and circulating immune complexes, phagocytal and functional-metabolic activity of neutrophils). The main lymphocyte subpopulations were identified on commercial kits of monoclonal antibodies to clusters CB3+, CB4+, CB8+-, CB16+, CB72+ (Russian-made) by indirect immunofluorescence on the microscope "LUMAM I-3": nonspecific functional activity of T-cells was studied by the leukocyte migration inhibition test (LMIT) for cell stimulation with PHA-P; concentration of the major classes of serum immunoglobulins (M, G, A) was identified by the raditional method of immunodiffusion in agar, using commercial sets of monospecific anti-immunoglobulin serum: concentration of the total IgE- by ELISA: circulating immune complexes - by the precipitation reaction in PEG-6000 followed by spectrophotometry of the precipitates: phagocytic activity of neutrophils was studied in the reaction of phagocytosis with latex particles and testing the nitroblue tetrazolium recovery (NBT test), while both spontaneous and pyrogenal-stumulated, parallel versions of both tests were carried out. An attempt to determine significant effects of the medical preparation on the immune response of the organism using average values did not yield a statistically significant effect (Table 2).

**Table 2**

| **The average group values of the examined immunological parameters during the study** | | | |
|---|---|---|---|
| **Immunological characteristics** | **Period of study** | | **P** |
| | Before | After | |
| CD3⁺ (%) | 49.2+1.7 | 51.6±1.1 | >0.05 |
| CD4⁺ (%) | 33.5+0.7 | 35.6±0.7 | >0.05 |
| CD8⁺ (%) | 21.6+1.3 | 22.7±0.8 | >0.05 |
| CD16⁺ (%) | 14.1+1.5 | 15.9+1.2 | >0.05 |
| CD72⁺ (%) | 16.5+1.5 | 17.1+1.3 | >0.05 |
| CD4⁺CD8⁺ | 1.62+0.11 | 1.58+0.11 | >0.05 |
| ITML | 0.63+0.03 | 0.61+0.13 | >0.05 |
| Ig M (g/l) | 0.63+0.08 | 0.98+0.07 | <0.05 |
| Ig G (g/l) | 9.19+0.83 | 9.66+0.66 | >0.05 |
| Ig A (g/l) | 1.48+0.20 | 1.57+0.29 | >0.05 |
| Ig E (ME/ml) | 73.07+2.26 | 60.15+5.49 | >0.05 |
| CIC (conv.units.) | 11.07+1.22 | 10.08+0.83 | >0.05 |
| NRT spont. (%) | 18.13+0.92 | 20.08+2.06 | >0.05 |
| NRT stim. (%) | 40.1+1.25 | 35.0+3.7 | >0.05 |
| Slnrt | 2.24+0.06 | 2.26+0.07 | >0.05 |
| PHAspont (%) | 18.4+0.36 | 19.0+0.76 | >0.05 |
| PHAstim (%) | 38.5+1.51 | 40. 6+1 D | >0.05 |
| Slpha | 2.24+0.1 | 2.16+0.07 | >0.05 |

The exception is serum immunoglobulin M class, which showed a statistically significant increase in iconcentration (0.63 + 0.08 g / L to 0.98 + 0.07 g / L).

To investigate the safety of the medical preparation «RENESSANS," it was administered to animals intraperitoneally in various doses both undiluted and diluted in a saline solution. The doses were selected based on the recommendations of the maximum allowable amount of fluid for injection into the peritoneal cavity, which for rats is 5 ml. The choice of the dose for injection was determined according to the classification of toxicity of chemicals in accordance with GOST (government standards) 12.1.007-76. The medical preparation was administered intraperitoneally once daily.

During the experiment, the rats were divided into control and experimental groups. There were four experimental groups with different doses and timing of the administration.

The medical preparation dissolved in a saline solution was administered intraperitoneally to the animals in reference experiments (Table 3).
1. The animals of the first group were injected intraperitoneally daily with 0.06 ml of the medical preparation + 3.0 ml of physiological solution, which exceeds the recommended therapeutic dose 1.7 times. The rats were sacrificed after 5, 10 and 15 days after the beginning of the experiment.
2. The animals of the second group were injected once by 0.66 ml of the medical preparation and 3.0 ml of physiological solution, which is 11 times higher than the recommended therapeutic dose, the animals, were sacrificed 5 days after the start of the administration.
3. The animals of the third group were injected once with 2 ml of the preparation and 3.0 ml of saline solution, which is 33 times higher than the recommended therapeutic dose and the animals were sacrificed 10 days after the beginning of the experiment.
4. The fourth group of animals was injected once with 5 ml of pure medical preparation without dilution, which exceeds the recommended therapeutic dose 83 times, and the animals were sacrificed 15 days after the beginning of the experiment.

The main data of the analysis of the morphological blood composition and differential white blood cell count are shown in Table 3.

**Table 3**

| **Morphologic composition of blood of experimental animals** | | | | | | |
|---|---|---|---|---|---|---|
| Blood index | Control | | Series of experiments | | | |
| | pure | with NaCl | I | II | III | IV |
| Erythrocytes 10 ⁹/l | 5.8+0.1 | 6.3+0.1 | 5.9+0.1 | 6.1+0.1 | 6.1+0.1 | 6.2+0.1 |
| Hbg/l | 116+2 | 118+2.5 | 105+3.0 | 117+3.0 | 114+3.0 | 115+3.0 |
| Color index | 0.9+0.01 | 0.9+0.01 | 0.85+0.01 | 0.90-0.01 | 0.90+0.01 | 0.92+0.01 |
| Segmented neutrophils 10 ⁹/l | 33+1.5 | 32+1.4 | 29+2.0 | 31+2.0 | 33+2.0 | 32+2.0 |
| Lymphocytes 10 ⁹/l | 54+1.2 | 60.5+1.2 | 54+1.0 | 58+1.5 | 55+2.0 | 58+2.0 |
| Monocytes 10 ⁹/l | 2+0.1 | 2.5+0.1 | 2.7+0.2 | 4.3+0.1 | 2.5+0.2 | 3.0+0.1 |
| Leukocytes 10 ⁹/l | 9.2±0.6 | 8.6±0.8 | 8.5±1.0 | 9.9±1.0 | 8.9±1.5 | 10.0±2 |
| CO 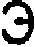 (MM. 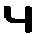 ) | 1.6+0.01 | 2+0.01 | 1.3+0.002 | 2.0+0.07 | 2.5+0.05 | 2.0+0.01 |

As seen from Table 3, the examined medication did not cause statistically significant changes in the blood count of the experimental animals compared to the control rats; therefore, the immunomodulatory medicine has no toxic effects on the blood system. Histological examinations of the organs of animals in the control and experimental groups showed that the medical preparation «RENESSANS" did not cause any changes in the histological structure of the internal organs of the experimental animals, which also confirms non-toxicity of the medical preparation.

Thus, the medical preparation «RENESSANS" has antibacterial and antiulcer effects with low toxicity and the possibility of its use in the injectable form.

## Claims

1. A medical preparation, exhibiting antibacterial, antiulcer, and immunomodulatory activity comprising iodine, potassium iodide, starch, glucose, ascorbic acid, sodium chloride and distilled water, wherein said preparation comprises the following components in the following ratio (wt%):
| | |
|---|---|
| Iodine | 0.4 - 2.0 |
| Potassium Iodide | 0.8 - 4.0 |
| Starch | 10.0 - 40.0 |
| Ascorbic acid | 0.4 - 2.0 |
| Glucose | 1.2 - 4.8 |
| Sodium chloride | 0.3 - 1.8 |
| Purified water | the rest. |

2. The preparation according to claim 1 for use in the treatment of mammals, including humans, of peptic ulcer disease.

3. The preparation for use according to claim 2 wherein said preparation is administered intravenously, intramuscularly or orally to a patient in a pharmaceutically effective amount.

## Patentansprüche

1. Pharmazeutisches Erzeugnis mit antibakterielle, Antiulkus- und immunomodulatorischer Wirkung, das Jod, Kaliumjodid, Stärke, Glukose, Ascorbinsäure, Natriumchlorid und destilliertes Wasser enthält,
**dadurch gekennzeichnet,**
**dass** dieses Erzeugnis die folgenden Komponenten in folgenden gewichtsprozentualen Mengen aufweist:
| | |
|---|---|
| Jod | 0,4-2,0 |
| Kallumjodid | 0,8-4,0 |
| Stärke | 10,0-40,0 |
| Askorbinsäure | 0,4-2,0 |
| Glukose | 1,2-4,8 |
| Natriumchlorid | 0,3-1,8 |
| Gereinigtes Wasser | Restmenge |

2. Pharmazeutisches Erzeugnis nach Anspruch 1,
**dadurch gekennzeichnet,**
**dass** es bei der Behandlung von Säugetieren, einschließlich von menschlichen Säugetieren, bei der Magengeschwürkrankheit angewandt wird.

3. Pharmazeutisches Erzeugnis zur Verwendeung nach Anspruch 2,
**dadurch gekennzeichnet,**
**dass** es einem Patienten intravenös, intramuskulär oder oral in einer pharmazeutisch wirksamen Menge eingeflößt wird.

## Revendications

1. Produit pharmaceutique ayant un effet antibactérien, anti-ulcère et immunomodulant qui contient de l'iode, de l'iodure de potassium, de l'amidon, du glucose, de l'acide ascorbique, du chlorure de sodium et de l'eau distillée, dont la caractéristique consiste en les composants ci-dessous en quantité proportionnellement pondérale suivante:
| | |
|---|---|
| Iode | 0,4-2,0 |
| Iodure de potassium | 0,8-4,0 |
| Amidon | 10,0-40,0 |
| Acide ascorbique | 0,4-2,0 |
| Glucose | 1,2-4,8 |
| Chlorure de sodium | 0,3-1,8 |
| Eau purifiée | Quantité résiduelle |

2. Produit pharmaceutique selon la revendication 1,
**caractérisé par** son application dans le traitement de l'ulcère gastrique chez les mammifères, notamment chez les êtres humains.

3. Produit pharmaceutique selon la revendication 2,
**caractérisé par** son utilisation lors de l'injection au patient par voie intraveineuse, intramusculaire ou orale en une quantité pharmaceutiquement active.
